# EUROPÄISCHE PATENTSCHRIFT

(11) **EP 2 339 992 B1**
(45) Veröffentlichungstag und Bekanntmachung des Hinweises auf die Patenterteilung: **01.04.2015**
(21) Anmeldenummer: 09740637.5
(22) Anmeldetag: 10.09.2009
(51) Int. Cl.: A61F 2/30, A61F 2/44

(54) **Gelenkimplantat**
Joint implant
Implant articulaire

(30) Priorität: 25.09.2008 DE 202008012749 U
(43) Veröffentlichungstag der Anmeldung: 06.07.2011
(73) Patentinhaber: Taurus GmbH & Co. KG, 63755 Alzenau (DE)
(72) Erfinder: BLEISTEIN, Frank, 63755 Alzenau (DE)
(74) Vertreter: Lippert, Stachow & Partner
(86) Internationale Anmeldenummer: PCT/DE2009/001283
(87) Internationale Veröffentlichungsnummer: WO 2010/034287

(56) Entgegenhaltungen:
- EP-A1- 1 857 079
- WO-A1-2005/007040
- WO-A2-2007/140382
- WO-A2-2008/088777
- US-A1- 2007 032 875
- US-A1- 2007 073 311
- US-A1- 2008 221 689

## Beschreibung

Die Erfindung betrifft ein Gelenkimplantat mit einem oberen und unteren Implantatteil, die mittels mindestens eines Gelenkes miteinander verbunden sind, wobei das obere Implantatteil mit seiner Oberseite und das untere Implantatteil mit einer Unterseite an jeweils gelenkig miteinander zu verbindenden Knochen anlegbar und abstützbar sind und wobei das obere und/oder das untere Implantatteil einen Teil des jeweiligen Gelenkes bereitstellende Gelenkbereiche aufweisen, und wobei ein Verbindungsmittel vorgesehen ist, welches das obere und das untere Implantatteil unter Ausbildung einer zusammenhängenden Baugruppe und unter Ermöglichung einer Gelenkbewegung gelenkig verbinden.

Derartige Gelenkimplantate werden beispielsweise als Bandscheibenprothesen eingesetzt, um benachbarte Wirbel einer Wirbelsäule gelenkig miteinander zu verbinden. Das Implantat kann hierbei ein oder mehrere Gelenkbereiche aufweisen, die unabhängig voneinander eine gelenkige Bewegung der jeweils angrenzenden Bereiche des Implantats zueinander ermöglichen, beispielsweise Verschwenk- oder Verkippbewegungen gegeneinander, wobei das Gelenk in Art eines Kardangelenks aufgebaut sein kann. Für den Implantationsvorgang hat es sich als zweckmäßig erwiesen, die einzelnen Implantatteile miteinander zu verbinden, beispielsweise durch geeignete Bolzen-, Zapfenverbindungen.

Andererseits ist es erforderlich, das Implantat an die jeweiligen anatomischen Verhältnisse anzupassen und gleichzeitig eine einfache Handhabung des Implantats im Vorfeld und/oder während einer Implantation zu ermöglichen. Hierbei kann es erforderlich sein, das Implantat an unterschiedlich große Knochen mit unterschiedlichen großen Implantatanlageflächen anzupassen, und/oder an unterschiedliche Abstände der Verbindungsbereiche der Knochen zueinander, beispielsweise wenn das Implantat an unterschiedlichen Wirbeln einer Wirbelsäule einzusetzen oder für Patienten verschiedener Größe und/oder Gewichtes einzusetzen ist, an unterschiedliche Gelenkbewegungen wie z.B. unterschiedliche Verschwenkwinkel in Abhängigkeit von den jeweiligen anatomischen Gegebenheiten und dergleichen.

Weiterhin soll das Implantat bei der Implantation sicher und einfach handhabbar sein und im implantierten Zustand eine hohe Lebensdauer aufweisen, insbesondere hohe mechanische Belastungen aushalten können.

Zudem soll das Implantat möglichst einfach aufgebaut sein, was sowohl der leichteren Herstellung als auch der einfacheren Handhabung des Implantats bei einer Sterilisation und/oder eine einfachere Integration in den Körper des jeweiligen Patienten gilt.

Die US 2007/032875 A1 beschreibt ein Implantat, bei welchem die Aufnahme des Anlageteils durch ein Abdeckelement abgedeckt ist, welches zur Befestigung in Richtung parallel zu der Implantatlängsachse an das Anlageteil heranzuführen ist.

Die WO 2007/140382 A2 beschreibt ein Implantat mit oberem und unterem Anlageteil, wobei eine Spiralfeder mit den beiden Anlageteilen verschraubt ist.

Die US 2008/221689 A1 beschreibt ein Implantat mit oberem und unterem Anlageteil, welche ohne Anordnung eines Verbindungselementes ein Gelenk ausbilden.

Die EP 1857079 A1 beschreibt ein Implantat, bei welchem ein Zapfen eines Anlageteils in die Unterseite des anderen Anlageteils eingreift.

Die WO 2005/007040 A1 beschreibt ein Implantat mit kalottenartigen Gelenkflächen und mit einem konvexen und einem konkaven Verbindungsteil, welche durch einen Bolzen miteinander verbunden sind, welcher in die Unterseite eines der Implantatteile eingreift.

Der Erfindung liegt somit die Aufgabe zugrunde, ein Gelenkimplantat zu schaffen, welches einfach an unterschiedliche anatomische Verhältnisse anpassbar ist, einfach handhabbar ist und eine hohe Lebensdauer im implantierten Zustand aufweist.

Erfindungsgemäß wird die Aufgabe wird durch ein Gelenkimplantat nach Anspruch 1 gelöst. Erfindungsgemäß weist das obere und/oder das untere Implantatteil ein dem jeweiligen Knochen zugewandtes, an diesem festlegbares Anlageteil auf, welches vorzugsweise plattenförmig ausgebildet ist, und ein an diesem befestigbares Gelenkteil, welches den Gelenkbereich des Implantatteils umfasst. Ferner ist das Verbindungsmittel sich im Wesentlichen parallel zur Implantatlängsachse erstreckend in einer knochenseitig offenen Aufnahme des Gelenkbereichs des oberen und/oder unteren Implantatteils gehaltert, wobei ein Abdeckelement vorgesehen ist, welches quer zur Implantatlängsachse verschiebbar ist und welches die Aufnahme zumindest teilweise abdeckt, wobei das Abdeckelement als an den Knochen anlegbares und das Implantat gegen den Knochen abstützendes Anlageteil ausgebildet ist.

Das obere und/oder untere Gelenkteil ist vorzugsweise als separates Bauteil ausgebildet, es ist zu dessen Befestigung an dem jeweiligen Anlageteil zumindest relativ zu diesem lageveränderlich. Hierdurch können der Gelenkbereich und der Anlagebereich des Implantatteils mit dem Knochen unabhängig voneinander ausgebildet und an die jeweiligen anatomischen Verhältnisse angepasst werden, wobei diese beiden Teile dann unter Ausbildung des jeweiligen Implantatteiles miteinander verbindbar sind.

Das Anlageteil kann hierdurch ferner eine durchgehende Anlagefläche für den Knochen bereitstellen, die beispielsweise nicht durch eine Trennlinie von Gelenk- und Anlageteil und/oder sonstige Durch- oder Unterbrechungen unterbrochen wird. Diese Trennlinie kann z. B. seitlich an dem Implantatteil angeordnet sein. Das Anlageteil kann sich somit durchgehend über die gesamte der Knochenanlagefläche zugewandte Implantatseite erstrecken. Anlageteil und Gelenkteil sind vorzugsweise entlang einer quer oder senkrecht zur Implantatlängsachse stehenden Fläche oder Ebene getrennt. Das Anwachsen des Implantatteils an den Knochen wird hierdurch erleichtert. Zudem kann hierdurch das Verbindungsmittel zwischen dem Gelenkteil und dem Anlageteil gehaltert sein, beispielsweise mit einer zwischen den beiden Teilen angeordneten kopfartigen Verbreiterung desselben. Allgemein kann der knochenseitige Bereich des Gelenkteils eine Aufnahme für das Verbindungsmittel aufweisen, die zu der dem Knochen zugewandten Seite hin geöffnet sein kann, so dass ein zum Knochen hin offener Bereich der Verbindungsmittelaufnahme durch das Anlageteil zumindest teilweise abgedeckt wird. Alternativ oder gleichzeitig kann das Verbindungsmittel mittels des Anlageteils an dem Gelenkteil fixiert oder gegen Lageveränderung oder Lösen gesichert werden. Da die Aufnahme des Verbindungsmittels an dem Gelenkteil von dem sich an dem Knochen abstützenden Anlageteil zumindest teilweise oder vollständig abgedeckt wird, ist das Implantat besonders sicher handhabbar und kann über einen langen Zeitraum implantiert sein. Vorzugsweise ist das Gelenkteil mit an diesem angeordneten Verbindungsmittel an dem Anlageteil des zugehörigen Gelenkteils befestigbar ist.

Das Verbindungsmittel kann zumindest zwei oder sämtliche Gelenkteile des Implantats durchgreifen, beispielsweise das obere und/oder untere Gelenkteil oder jeweils zwei benachbarte und ggf. miteinander gelenkig zusammenwirkende Gelenkteile des Implantats. Das Verbindungsmittel kann die jeweiligen Gelenkteile in Implantatlängsachse Zugkraft aufnehmend aneinander sichern.

Bevorzugt ist das jeweilige Gelenkteil formschlüssig an dem zugeordneten Anlageteil des oberen und/oder unteren Implantatteils befestigt, insbesondere durch eine Rastverbindung, wobei die Formschlussverbindung vorzugsweise lösbar ist. Gegebenenfalls können Gelenkteil und Anlageteil des jeweiligen Implantatteils auch dauerhaft miteinander verbunden werden, beispielsweise durch Verkleben oder Verschweißen. Allgemein ist das an dem Anlageteil befestigte Gelenkteil gegenüber diesem lagefixiert und zusammen mit dem Anlageteil gegenüber dem anderen Gelenkteil bewegbar.

Das Gelenk- und Anlageteil sind vorzugsweise zwangsgeführt in ihre Sollposition zueinander überführbar, in welcher diese dauerhaft miteinander zu verbinden sind. Hierzu können an Gelenk- und Anlageteil jeweils miteinander korrespondierende Führungselemente vorgesehen sein.

Vorzugsweise sind das obere und/oder untere Gelenkteil um die Implantatlängsachse bzw. um die Verbindungsmittellängsachse gegeneinander verdrehbar. Das Verbindungsmittel kann sich somit allgemein zumindest im Wesentlichen in Implantatlängsachse erstrecken.

Vorzugsweise ist das Verbindungsmittel relativ zu dem oberen und/oder unteren Gelenkteil quer zur Implantatlängsachse verschiebbar ausgebildet. Hierdurch ist eine Gelenkverbindung entsprechend den jeweiligen anatomischen Erfordernissen möglich. Die Verschiebbarkeit der Bauteile zueinander kann durch geeignete Mittel an dem Gelenkteil ermöglicht werden, wie z.B. durch Anordnung eines Langloches an diesem, diese Mittel können auf das Gelenkteil beschränkt sein, so dass eine entsprechende Ausgestaltung des Anlageteils nicht erforderlich ist. Durch das Anlageteil können diese eine Verschiebung ermöglichenden Mittel an dem Gelenkteil zum jeweils benachbarten Knochen hin teilweise oder vollständig abgedeckt werden.

Vorzugsweise sind das Anlageteil und das diesem zugeordnete Gelenkteil durch Formschlussmittel, besonders bevorzugt durch Rastmittel, miteinander befestigbar, gegebenenfalls können die beiden Teile auch dauerhaft miteinander verbunden sein, beispielsweise durch eine Stoffschlussverbindung wie Verkleben oder Verschweißen. Diese Verbindung kann nach der Halterung des Verbindungsteils an dem Gelenkteil erfolgen. Vorzugsweise sind das Anlage- und das Gelenkteil lösbar miteinander verbunden, ggf. auch unlösbar. Dies gilt jeweils für zumindest eines oder vorzugsweise für beide Implantatteile.

Das Gelenkteil kann im Wesentlichen plattenförmig ausgebildet sein und sich vorzugsweise flächig an dem korrespondierenden Anlageteil abstützen. Gelenkteil und Anlageteil sind vorzugsweise ortsfest und nicht lageveränderlich aneinander befestigt. Das Gelenkteil kann sich im Wesentlichen über die gleich Breite und/oder Länge erstrecken wie das Anlageteil, jeweils bezogen auf die Hauptebene derselben, gegebenenfalls bis auf seitliche Befestigungsbereiche an einem der beiden Bauteile, die der Befestigung der beiden Bauteile aneinander dienen können.

Das Anlageteil und das korrespondierende Gelenkteil des jeweiligen Implantatteils können derart ausgebildet sein, dass diese zumindest im Wesentlichen durch Verschieben quer zur Implantatslängsachse miteinander ankoppelbar sind. Allgemein kann die Befestigungsrichtung zur Festlegung von Anlage- und Gelenkteil aneinander somit verschieden sein von der Hauptbelastungsrichtung des Implantats und/oder der Implantatslängsachse, die in Verbindungsrichtung der gelenkig miteinander zu verbindenden Knochen liegt. Hierzu können das Anlageteil und/oder das Gelenkteil eine Axialführung aufweisen, entlang deren die beiden Bauteile aneinander geführt in ihre Sollposition zueinander überführbar verschiebbar sind. Weiterhin kann an dem Anlage- und/oder Gelenkteil ein Anschlag vorgesehen sein, welcher die Sollposition der beiden Teile zueinander definiert.

Vorzugsweise sind Anlageteil und das korrespondierende Gelenkteil aneinander lösbar befestigt, wobei Anlage- und Gelenkteil durch Verriegelungsmittel in ihrer Sollposition zueinander festgelegt werden können. Die lösbaren Befestigungsmittel sind vorzugsweise von zumindest von einer der Seitenflächen der beiden Teile her zugänglich und lösbar, d.h. aus einer Richtung quer oder im Wesentlichen senkrecht zu der Implantatlängsachse, beispielsweise von der dorsalen und/oder lateralen Seitenfläche her. Vorzugsweise ist die Verbindung von Gelenkteil und Anlageteil durch Angriff eines Werkzeugs an die Gelenkteilseite her möglich, welche von der Knochenanlagefläche des Anlageteils verschieden ist.

Das Anlageteil, gegebenenfalls auch das Gelenkteil, kann zur Befestigung an dem jeweils anderen Bauteil eine taschenartige Aufnahme für das korrespondierende Bauteil aufweisen, welche mit einem Teilbereich das korrespondierende Bauteil zumindest teilweise überbrückt. Der Überbrückungsbereich kann als Befestigungsbereich ausgebildet sein, der mit einem Befestigungselement des korrespondierenden Bauteils zusammenwirken kann. Hierbei kann ein Rastelement des Gelenkteils an dem Überbrückungsbereich des Anlageteils festgelegt sein, ggf. kann der Überbrückungsbereich auch an dem Gelenkteil und das mit diesem zusammenwirkende Befestigungselement bzw. Rastelement an dem Anlageteil angeordnet sein. Allgemein kann die taschenartige Aufnahme des jeweiligen Teils seitlich in eine Führung, insbesondere Axialführung, übergehen, entlang welcher das jeweils korrespondierende Bauteil in seine Sollposition überführbar ist, um Anlageteil und korrespondierendes Gelenkteil zu befestigen. Das Gelenk- und Anlageteil können aneinander in Implantatlängsachse übergreifende Teilbereiche aufweisen, beispielsweise in Art einer Schwalbenschwanzführung oder ähnlichen Hinterschneidung, um in dieser Richtung hohe Zugkräfte aufnehmen zu können.

Vorzugsweise ist das Verbindungsmittel aus zumindest zwei Elementteilen ausgebildet, die unter Ausbildung einer Verbindung in Implantatlängsachse lösbar oder vorzugsweise dauerhaft miteinander befestigbar sind, beispielsweise durch eine Klebe- und/oder Schweißverbindung. Eines oder beide der Verbindungsmittelteile kann von der dem jeweiligen Knochen zugewandten Seite des Gelenkelementes her durch eine Durchtrittsöffnung desselben durchgeführt werden, so dass die dem jeweils benachbarten Element zugewandten Bereiche, die gegebenenfalls aus dem jeweiligen Gelenkelement in Richtung auf das jeweils andere Bauteil heraustreten können, miteinander verbindbar sind. Allgemein kann die Verbindungsmittelaufnahme des Gelenkelementes eine Hinterschneidung bzw. Querschnittsverengung aufweisen, welche von dem beispielsweise kopfartig verbreiterten Befestigungsbereich des Verbindungsmittels oder eines Teils desselben hintergriffen werden kann.

Die Befestigung des Verbindungsmittels an den beiden Implantatteilen, insbesondere den Gelenkelementen oder ggf. auch den Anlageteilen derselben, kann vorzugsweise unlösbar erfolgen, beispielsweise durch eine unlösbare Verbindung der beiden Verbindungsmittelteile, wodurch eine zusammenhängende Gelenkbaugruppe geschaffen werden kann. Hierbei können einer oder beide Endbereiche des Verbindungsmittels gegenüber dem jeweiligen Implantatteil bzw. Gelenkelement lageveränderlich sein, beispielsweise verdrehbar und/oder diesem gegenüber querverschiebbar, beispielsweise durch die oben beschriebene Anordnung der jeweilige Endbereich des Verbindungsmittels an einem Langloch des Implantat- bzw. Gelenkteils. Der Verbindungsbereich der beiden Teile des Verbindungsmittels kann allgemein in Art einer Bolzen- Zapfenverbindung ausgebildet sein. Unter Umständen kann dies jedoch auch ein Endbereich des Verbindungsmittels lageveränderlich an einem der Gelenkteile befestigt sein, z.B. durch eine Formschlussverbindung, wobei der gegenüberliegende Endbereich ortsfest an dem weiteren zusammenwirkenden Gelenkteil befestigt ist.

Der Verbindungsbereich der beiden Elementteile des Verbindungsmittels kann von der dem Anlageteil zugewandten Seite des Gelenkelementes her zugänglich sein, wobei der Verbindungsbereich bei demontierten Anlageteil von dem Gelenkteil nach außen vorstehen kann oder in einer zu dem jeweiligen Anlageteil hin offenen Ausnehmung angeordnet ist und somit durch Eingriff in die Ausnehmung zugänglich ist. Das Verbindungswerkzeug kann somit in Implantatlängsachse an die Verbindungsstelle herangeführt werden. Es können bei demontiertem Anlageteil die beiden Elementteile dauerhaft miteinander verbunden werden, beispielsweise durch Verschweißen, insbesondere durch Laserschweißen. Nach dem dauerhaften Verbinden der beiden Elementteile kann dann der Anlageteil an dem Gelenkelement befestigt werden. Das Anlageteil kann die Aufnahme des Gelenkteils für das Verbindungsmittel teilweise oder vollständig abdecken. Der Befestigungsbereich des Verbindungsmittels in dem Gelenkteil von der Gelenkteilaufnahme und dem an diesem festgelegten Anlageteil zumindest im Wesentlichen vollständig abgekapselt sein kann. Das Anlageteil und das zugehörige Gelenkteil können somit das Verbindungsmittel vorzugsweise unverlierbar zwischen sich haltern.

Vorzugsweise ist das Verbindungsmittel derart an den beiden Implantatteilen bzw. den Gelenkelementen derselben festgelegt, dass die beiden Implantatteile mit Spiel in Implantatlängsachse bzw. in Längsachse des Verbindungsmittels aneinander festgelegt sind. Hierdurch können die beiden Implantatteile gegeneinander oder gegen ein zwischen den beiden Implantatteilen angeordnetes Zwischenteil eine Verschwenk-, Abwälz- oder Verkippbewegung durchführen, wobei bei dieser Gelenkbewegung die beiden Implantatteile miteinander oder jeweils mit dem Zwischenteil in währender Anlage sind. Hierbei kann das Verbindungsmittel mit einer kopfartigen Verbreiterung in einer Aufnahme des jeweiligen Implantat- bzw. Gelenkteils angeordnet sein. Durch das Spiel ist somit zugleich eine Relativbewegung des Kopfes zu der Aufnahme ermöglicht. Gegebenenfalls können die Implantatteile durch das Verbindungsmittel auch in Implantatlängsachse spielfrei miteinander befestigt sein, wenn der Befestigungsbereich von Verbindungsmittel und Gelenkteil entsprechend ausgebildet ist, z.B. mit entsprechenden Krümmungsradien, was aber konstruktiv aufwändig ist.

Das Implantat kann ein oberes und unteres Gelenkteil aufweisen, welche unter Ausbildung einer Gelenkverbindung unmittelbar miteinander zur Anlage kommen bzw. miteinander wechselwirken. Die beiden Gelenkteile können hierbei in Art eines Kugelgelenkes gegeneinander verschwenkbar sein oder eine Abwälz- oder Verkippbewegung gegeneinander durchführen. Vorzugsweise ist ein Zwischenbauteil zwischen den Gelenkteilen des oberen und des unteren Implantatteils vorgesehen, welches jeweils gelenkig mit dem Gelenkteil des oberen und unteren Implantatteils wechselwirkt. Das Zwischenbauteil kann somit gegenüber dem oberen und und/oder unteren Gelenkteil (d.h. gegenüber dem Gelenkteil des oberen und/oder des unteren Implantatteils) gelenkig lageveränderlich sein, beispielsweise durch eine Verschwenkbewegung (z.B. in Art eines Kugelgelenkes), eine Abwälzbewegung oder eine Verkippbewegung. Das Zwischenbauteil kann hierbei jeweils als im wesentlichen plattenförmiges Bauteil ausgeführt sein, die den jeweiligen Gelenkteilen zugewandten Oberflächen des Zwischenbauteils können ggf. auch eine andere Querschnittskontur aufweisen und beispielsweise gewölbt ausgebildet sein, z.B. konkav oder konvex gewölbt. Vorzugsweise sind zumindest eine oder beide den benachbarten Gelenkteilen zugewandten Oberflächen des Zwischenbauteils eben ausgebildet, vorzugsweise zumindest im Wesentlichen planparallel. Die ebenen Oberflächen des Zwischenbauteils können zumindest im Wesentlichen senkrecht zu der Verbindungsachse der beiden Implantatteilen angeordnet sein. Die Gelenkoberflächen der Gelenkteile können zumindest teilweise in Form von Kugel- oder Zylinderabschnitten ausgebildet sein. Bei Ermöglichung einer Abwälzbewegung können sich an einen mittleren Zylinderabschnitt ebene Bereiche anschließen, so dass unter Begrenzung der Abwälzbewegung flächige Anlagebereiche der Gelenkteile miteinander oder jeweils mit einem Zwischenteil gegeben sind. Entsprechend können bei Ermöglichung einer Verkippbewegung sich an eine mittlere Verkippkante ebene Bereiche anschließen, so dass unter Begrenzung der Kippbewegung flächige Anlagebereiche der Gelenkteile miteinander oder jeweils mit einem Zwischenteil gegeben sind. Entsprechendes kann für eine Verschwenkbewegung gelten. Die Befestigungsbereiche von Gelenkelement und Anlageteil, z.B. die zusammenwirkenden Rastmittel derselben, sind hierbei vorzugsweise radial und/oder axial außerhalb dieser Anlagebereiche angeordnet.

Durch die Zusammenwirkung des oberen und unteren Implantatteils bzw. Gelenkteils mit dem Zwischenbauteil können jeweils zwei Gelenkbereiche des Implantats ausgebildet werden. Vorzugsweise ermöglichen die beiden Gelenkbereiche eine Verschwenk- Abwälz- oder Verkippbewegung in verschiedenen Richtungen, die einen Winkel miteinander einschließen können, z.B. einen Winkel von 90°. Dies gilt vorzugsweise für die Neutralstellung der beiden Implantateile bzw. Gelenkteile zueinander. Die Anlageteile der beiden Implantatteile sind vorzugsweise nicht kongruent ausgebildet, sonder derart, dass diese in derselben Richtung an den Gelenkteilen befestigbar sind, beispielsweise durch Verschiebung in der Hauptebene der Implantatteile bzw. der Anlageteile, und in dieser Stellung dann die Verschwenk- Abwälz- oder Verkipprichtungen der Implantatteile einen Winkel einschließen, vorzugsweise von 90°. Vorzugsweise sind die beiden Implantatteile um die Implantatlängsachse gegeneinander verdrehbar ausgebildet, so dass auch die Verschwenk-, Abwälz- oder Verkipprichtungen der beiden Implantatteile unterschiedliche Winkel zueinander einschließen können.

Vorzugsweise wird das Zwischenbauteil, welches insbesondere in Art einer Scheibe ausgebildet sein kann, von dem Verbindungsmittel durchgriffen, um das obere und untere Implantatteil bzw. die diesen zugeordneten Gelenkelemente miteinander zu verbinden. Das Verbindungsmittel kann hierbei in Verbindungsmittellängs- und/oder -querrichtung unverschiebbar oder lagefixiert an dem Zwischenteil angeordnet sein. Besteht das Verbindungsmittel aus zwei Teilen, so können diese jeweils an dem Zwischenteil, insbesondere den den Gelenkteilen zugewandten Oberflächen desselben, anliegen, vorzugsweise mit entsprechenden Anlageflächen, und das Zwischenteil hierdurch umschließen.

Das obere und/oder untere Implantatteil und/oder das zwischen diesen angeordnete Zwischenbauteil können zumindest im Wesentlichen als plattenförmige Bauteile ausgebildet sein. Insbesondere können die den jeweiligen Knochen zugewandten Anlageflächen des oberen und/oder unteren Implantatteils zumindest im wesentlichen durchgehend und geschlossen ausgebildet sein, so dass insbesondere ein Einwachsen von Knochenmaterial hin das Anlageteil oder die Aufnahme des Verbindungsmittels in dem Gelenkelement und/oder dem Zwischenbauteil, verhindert ist. Die Anlageflächen der Anlageteile können jedoch strukturiert ausgebildet sein, um ein Anwachsen von Knochenmaterial zu fördern. Die Erfindung wird nachstehend anhand eines Ausführungsbeispiels beschrieben und anhand der Figuren erläutert. Es zeigen:
- Figur 1: eine Explosionsdarstellung eines erfindungsgemäßen Implantats,
- Figur 2: eine Querschnittsdarstellung eines Implantats nach Figur 1,
- Figur 3: eine Seitenansicht des Implantats nach Figur 2.

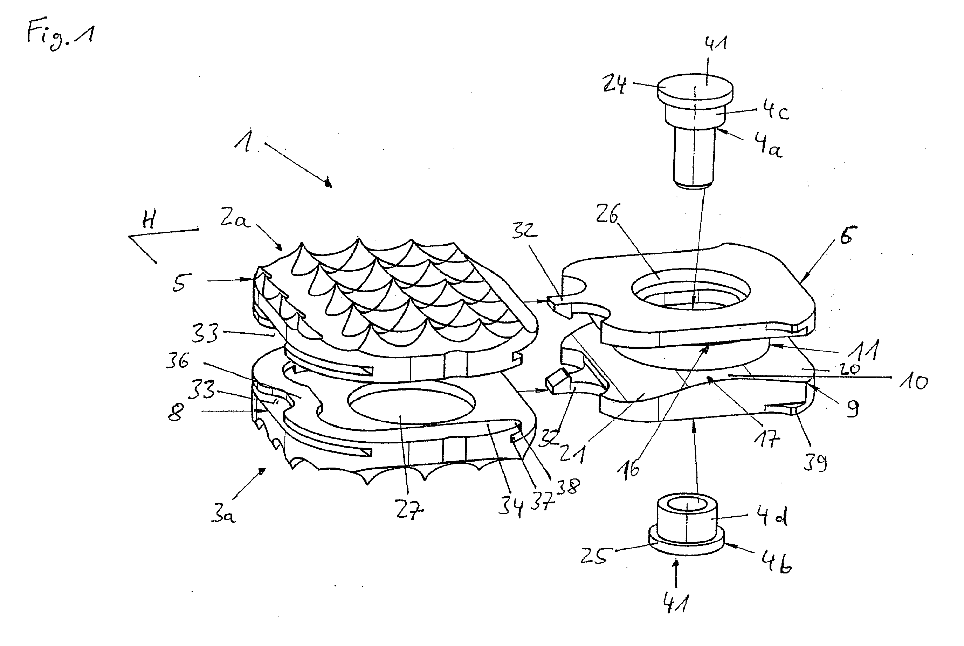

Die Figuren 1-3 zeigen eine Ausführungsform eines erfindungsgemäßen Gelenkimplantats 1 mit einem oberen und einem unteren Implantatteil 2, 3, wobei das obere Implantatteil 2 mit einer Oberseite 2a und das untere Implantatteil 3 mit seiner Unterseite 3a jeweils an Anlagebereiche von Knochen, insbesondere die Grundplatten angrenzender Wirbelkörper, anlegbar sind, um diese gelenkig miteinander zu verbinden. Weiterhin ist ein Verbindungsmittel 4 vorgesehen, welches die beiden Implantatteile 2,3 unter Ausbildung einer zusammenhängenden Baugruppe unter Ermöglichung einer Gelenkbewegung derselben miteinander verbindet. Das obere Implantatteil 2 besteht hierbei aus einem Anlageteil 5, welches die an dem Knochen anlegbare Oberseite 2a des Implantatteils aufweist, und einem an dem Anlageteil 5 befestigten, vorzugsweise separaten Gelenkteil 6, welches den Gelenkbereich 7 des oberen Implantatteils bereit stellt. Das untere Implantatteil 3 weist entsprechend ein die Unterseite 3a bereit stellendes Anlageteil 8 sowie ein an diesem befestigtes, vorzugsweise separates Gelenkteil 9 auf, welches den Gelenkbereich 10 des unteren Implantatteils bereit stellt. Die Verbindung der Implantat- und Gelenkteile kann jeweils lösbar sein.

Prinzipiell können die beiden Gelenkbereiche 7,10 des oberen und unteren Implantatteils unmittelbar miteinander unter Ausbildung einer Gelenkverbindung zusammenwirken. Nach dem Ausführungsbeispiel ist zwischen den beiden Implantatteilen 2,3 jedoch ein Zwischenteil 11 vorgesehen, welches eine dem oberen und unteren Anlageteil 5,8 zugewandte Ober- und Unterseite 12,13 aufweist, welche jeweils mit den Gelenkoberflächen 16,17 der Gelenkbereiche 7,10 unter Anlage an dieselben zusammenwirken, um so zwei Gelenke 14,15 des Implantats auszubilden. Die Ober- und Unterseite 12,13 des Zwischenteils 11 sind hierbei eben ausgeführt und vorzugsweise planparallel zueinander angeordnet. Die Gelenkoberflächen 16, 17 können jeweils gegenüber den Oberflächen des Zwischenteils 11 abwälzen, wozu die Gelenkoberflächen 16,17 jeweils mit einem mittigen gewölbten, vorzugsweise kreisbogenförmig gewölbten, Abwälzbereichen als Gelenkbereichen versehen sind.

Seitlich an die die Gelenkbewegung ermöglichenden Bereiche, hier die als Abwälzbereiche ausgebildeten Gelenkbereiche 7, 10, schließen sich jeweils vorzugsweise eben ausgebildete Anlagebereiche 20,21 an, die an dem oberen und unteren Gelenkteil vorgesehen sind, und die vorzugsweise eine flächige Anlage an die Ober- und Unterseite des Zwischenteils ermöglichen und zugleich die Gelenkbewegung begrenzen. Die Anlagebereiche 20,21 des jeweils oberen und unteren Implantatteil können in der Neutralstellung dieser Teile, d.h. bei senkrecht zur Implantatlängsachse angeordneten Anlageflächen, jeweils eine unterschiedliche Neigung zu der Implantatlängsachse 22 aufweisen, welche zugleich mit der Längsachse 23 des Verbindungsmittels 4 übereinstimmen kann. Das obere und untere Gelenkteil können sich hierbei im Hinblick auf die Ausbildung der Gelenkbereiche unterscheiden, beispielsweise im Hinblick auf die Wölbungs- oder Krümmungsradien der mittleren Abwälzbereiche und/oder die Neigung der sich seitlich anschließenden Anlagebereiche 20,21 zur Implantatlängsachse. Die Ausgestaltung erfolgt gemäß den anatomischen Erfordernissen. Das obere und untere Anlageteil 5, 8 können allgemein und unabhängig von dem Ausführungsbeispiel baugleich ausgeführt sein. Die einzelnen Bauteile, d.h. das obere und untere Anlageteil 5, 8, das obere und untere Gelenkteil 6,9 und das Zwischenteil 11 können im Wesentlichen als plattige Bauteile ausgebildet sein.

Das obere und untere Implantatteil 2,3 sind gegeneinander und gegen das Zwischenteil um die Verbindungsmittellängsachse 23 verdrehbar ausgebildet. Die beiden Implantatteile 2,3 können derart angeordnet sein, dass diese jeweils eine Bewegung wie z.B. eine Abwälz-, Verschwenk- oder Verkippbewegung um eine Achse durchführen, wobei die beiden Achsen einen Winkel von 90° zueinander und vorzugsweise auch zu der Implantatlängsachse einschließen können.

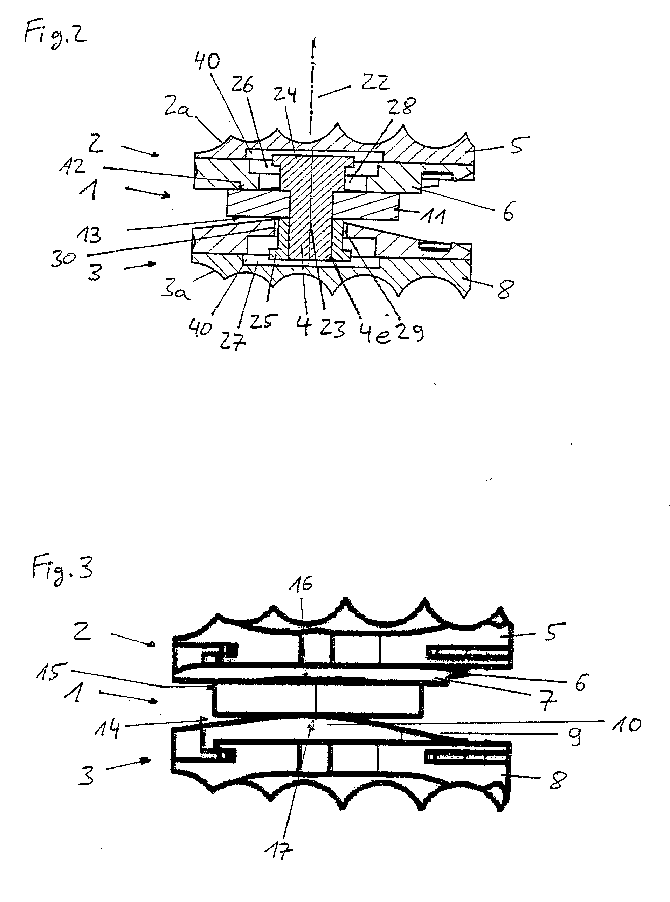

Das Verbindungsmittel 4 ist nach dem Ausführungsbeispiel zweiteilig mit einem Bolzen 4a und einem Aufnahmezapfen bzw. Aufnahmehülse 4b ausgebildet. Diese liegen im befestigten Zustand jeweils mit ihren kranzförmigen Anlagebereichen 4c, 4d an Ober- und Unterseite 12,13 des Zwischenteils 11 an und umschließen dieses spielfrei. Der Bolzen 4a und die Aufnahmehülse 4b können an dem stirnseitigen Verbindungsbereich 4e (siehe Fig. 2) dauerhaft miteinander verbunden sein, beispielsweise durch Laserschweißen. Die Verbindung wie z.B. Laserbestrahlung strahl kann bei zumindest teilweise oder vollständig demontiertem Anlageteil erfolgen, die Verbindung kann zumindest im Wesentlichen aus der Längsrichtung des Verbindungsmittels bzw. der Implantatlängsachse her erfolgen.

Die kopfartig erweiterten Verbindungsbereiche 24, 25 des Verbindungsmittels 4 sind hierbei in Aufnahmen 26, 27 der beiden Gelenkteile angeordnet, wobei die Aufnahmen mit Langlöchern 28,29 versehen sind, die eine laterale Verschiebung der beiden Implantatteile 2, 3 Verbindungsmittellängsachse ermöglichen. Die Langlöcher erstrecken sich hier in Längsrichtung der die Gelenkbewegung ermöglichenden Bereiche 18, 19 (die hier als Abwälzbereiche ausgeführt sind). Bei dieser Abwälzbewegung kann zugleich eine Art "Rutschen" des Abwälzbereiches über die jeweilige Oberfläche des Zwischenteils 11 erfolgen, was dadurch bewirkt werden kann, dass die inneren Seitenwände der Verbindungsmittelaufnahme (bzw. die inneren Seitenwände der Langlöcher entlang deren Endbereichen in Längsrichtung; siehe Bezugsziffer 30, entsprechend beim oberen Implantatteil um 90° verdreht) bei einer Verschwenk- bzw. Abwälzbewegung des Anlageteils gegenüber dem Zwischenteil zu einem unterschiedlichen Zeitpunkt mit dem Verbindungsmittel 4 zur Anlage kommen, beispielsweise, da die beiden Anlagebereiche 20,21 eine unterschiedliche Neigung aufweisen oder sich unterschiedlich weit in Richtung auf die mittlere Zone des jeweiligen Gelenk- bzw. Abwälzbereichs 18,19 hin erstrecken. Auch ein derartiges "Rutschen", welches mit einer Abwälzbewegung überlagert sein kann, sei hier noch als "abwälzen" verstanden, gegebenenfalls kann jedoch auch eine Abwälzbewegung ohne "Rutschen" realisiert sein.

Die Anlageteile 5,8 sind jeweils lösbar mit den zugeordneten Gelenkteilen 6,9 verbunden. Die Anlageteile 5,8 sind jeweils durch Formschlussmittel, insbesondere durch Rastmittel, mit den Gelenkteilen 6,9 verbunden. Hierzu weisen die Gelenkteile 6,9 Rastvorsprünge 32 auf, welche mit entsprechenden Rastaufnahmen 33 der Anlageteile 5 zusammenwirken. Es können somit wenn die Anlageteile mit den Knochen verwachsen sind, bei Bedarf die Gelenkteile von den Anlageteilen entkoppelt bzw. gelöst werden. Die Gelenkteile 6,9 können durch Verschiebung quer oder senkrecht zur Implantatlängsachse 22, vorzugsweise in der Hauptebene H der Anlageteile 5, verschoben werden, um an diesen festgelegt zu werden. Hierzu sind die Anlageteile 5 mit Axialführungen 34 versehen, welche seitlich an den Gelenkteilen 6,9 bei der Befestigungsbewegung der Gelenkteile anliegen können. Die Befestigungsmittel bzw. Rastaufnahmen 33 der Anlageteile sind hierbei in einer taschenartigen Aufnahme 35 angeordnet, welche einen frontalen Bereich der Gelenkteile 6,9 oder einen in Einschubrichtung vorderen Bereich der Seitenbereiche der Gelenkteile übergreifen können. Die Rastzungen 32 der Gelenkteile 6,9 untergreifen hierbei den überbrückenden Bereich 36 der taschenartigen Aufnahme. Die Einschubbewegung der Gelenkteile kann durch Anschläge begrenzt werden, hierzu können der überbrückende Bereich 36 und/oder die stirnseitigen Enden 37 der Axialführungen 34 als Anschläge für die Gelenkteile dienen, um deren Sollposition zu bestimmen. Aufgrund der Ausgestaltung der Rastverbindungen können diese durch ein Werkzeug unter Angriff aus einer Richtung im Wesentlichen quer oder senkrecht zu der Implantatlängsachse 22 entrastet werden. Durch die Hinterschnitte 38 an den Anlageteilen, die hier an den Axialführungen 34 vorgesehen sind, genauer gesagt an den beiden Endbereichen derselben, werden Teilbereiche der Gelenkteile 6,9 übergriffen, beispielsweise die Rastzungen 32 und/oder die seitlich vorspringenden Bereiche 39, die hier an den den Rastzungen gegenüberliegenden Endbereiche der Gelenkteile vorgesehen sind. Durch diese Formschlussmittel kann die Verbindung zwischen den Anlage- und Gelenkteilen hohe Zugkräfte in Implantatlängsachse aufnehmen.

Die Anlageteile überdecken in ihrer Sollposition die Befestigungsbereiche des Verbindungsmittels 4 und die Aufnahmen an den Gelenkteilen für eben diese Befestigungsbereiche zu den jeweiligen Knochen teilweise oder vorzugsweise zumindest im Wesentlichen vollständig, so dass für die Knochen jeweils eine durchgehende Anlagefläche bereit gestellt wird. Die Anlageteile können hierbei in ihrem mittleren Bereich eine gewisse Vertiefung 40 aufweisen (siehe Fig. 2), so dass ein Spiel zwischen den stirnseitigen Enden 41 (siehe Fig.1) des Verbindungsmittels 4 und den Innenflächen der Anlageteile resultiert und somit eine gelenkige Bewegung der Implantatteile gegenüber dem Zwischenteil ermöglicht ist.

## Patentansprüche

1. Gelenkimplantat mit einem oberen und einer unteren Implantatteil (2,3), die mittels mindestens eines Gelenkes miteinander verbunden sind, wobei das obere Implantatteil (2) mit einer Oberseite (2a) und das untere Implantatteil (3) mit einer Unterseite (3a) jeweils an gelenkig miteinander zu verbindenden Knochen anlegbar und abstützbar sind, wobei das obere und/oder das untere Implantatteil (2,3) jeweils einen Teil des Gelenkes bereitstellende Gelenkbereiche (7,10) aufweisen, und wobei ein Verbindungsmittel (4) vorgesehen ist, welches das obere und das untere Implantatteil (2,3) unter Ausbildung einer zusammenhängenden Baugruppe und unter Ermöglichung einer Gelenkbewegung miteinander verbindet, **dadurch gekennzeichnet**,
dass das obere und/oder das untere Implantatteil (2,3) jeweils ein dem jeweiligen Knochen zugewandtes, an diesem festlegbares Anlageteil (5,8) aufweist, welches die Ober- bzw. Unterseite des Implantats bereitstellt, und ein an dem Anlageteil (5,8) befestigbares Gelenkteil (6,9), welches den Gelenkbereich (7,10) des Implantatteils umfasst, unddass das Verbindungsmittel (4) sich im Wesentlichen parallel zur Implantatlängsachse (22) erstreckend in einer knochenseitig offenen Aufnahme (26,27) des Gelenkbereichs (7,10) des oberen und/oder unteren Implantatteils (2,3) gehaltert ist, und dass ein Abdeckelement vorgesehen ist, welches quer zur Implantatlängsachse (22) verschiebbar ist und welches die Aufnahme (26,27) zumindest teilweise oder zumindest im Wesentlichen vollständig abdeckt, und dass das Abdeckelement als an den Knochen anlegbares und das Implantat gegen den Knochen abstützendes Anlageteil (5,8) ausgebildet ist.

2. Implantat nach Anspruch 1, **dadurch gekennzeichnet, dass** das Verbindungsmittel (4) das obere und/oder untere Gelenkteil (6,9) durchgreift und diese in Implantatlängsachse (22) Zugkraft aufnehmend sichert.

3. Implantat nach Anspruch 1 oder 2, **dadurch gekennzeichnet, dass** das Gelenkteil (6,9) formschlüssig an dem Anlageteil (5,8) des oberen und/oder unteren Implantatteils (2,3) befestigt ist.

4. Implantat nach einem der Ansprüche 1-3, **dadurch gekennzeichnet, dass** das Verbindungsmittel (4) relativ zu dem oberen und/oder unteren Anlageteil (5,8) und/oder Gelenkteil (6,9) quer zur Implantatlängsachse (22) verschiebbar ist.

5. Implantat nach einem der Ansprüche 1-4, **dadurch gekennzeichnet, dass** das Verbindungsmittel (4) zwei Teile (4a,4b) aufweist, die unter Ausbildung einer Verbindung in Implantatlängsachse (22) dauerhaft miteinander befestigbar oder befestigt sind.

6. Implantat nach einem der Ansprüche 1-5, **dadurch gekennzeichnet, dass** das Implantatteil (2,3) und das diesem zugeordnete Gelenkteil (6,9) durch Formschlussmittel (32,33) miteinander befestigbar sind.

7. Implantat nach einem der Ansprüche 1-6, **dadurch gekennzeichnet, dass** das obere und/oder untere Gelenkteil (6,9) plattenförmig ausgebildet ist und sich an dem Anlageteil (5,8) abstützt.

8. Implantat nach einem der Ansprüche 1-7, **dadurch gekennzeichnet, dass** das obere und/oder untere Anlageteil (5,8) und das zugeordnete Gelenkteil (6,9) jeweils derart aneinander befestigbar sind, dass diese zumindest im Wesentlichen durch Verschieben quer zur Implantatlängsachse aneinander ankoppelbar sind.

9. Implantat nach dem Anspruch 8, **dadurch gekennzeichnet, dass** das Anlageteil (5,8) und/oder das Gelenkteil (6,9) eine Axialführung aufweisen, entlang deren die beiden Bauteile aneinander geführt in ihre Sollposition zueinander verschiebbar sind.

10. Implantat nach einem der Ansprüche 1-9, **dadurch gekennzeichnet, dass** das Anlageteil (5,8) oder das Gelenkteil (6,9) ein an dem jeweils anderen Bauteil festlegbares Befestigungsmittel aufweist und dass der Befestigungsbereich eines der beiden Bauteile das jeweils andere zumindest teilweise unter Ausbildung einer taschenartigen Aufnahme überbrückt.

11. Implantat nach einem der Ansprüche 1-10 **dadurch gekennzeichnet, dass** das Anlageteil (5,8) und das zugehörige Gelenkteil (6,9) derart ausgebildet sind, dass diese bei Befestigung des Gelenkteils (6,9) in seiner Sollposition an dem Anlageteil (5,8) das Verbindungsmittel (4) unverlierbar haltern.

12. Implantat nach einem der Ansprüche 1-11, **dadurch gekennzeichnet, dass** zwischen dem Gelenkteil (6,9) des oberen und des unteren Implantatteils (2,3) ein Zwischenbauteil (11) vorgesehen ist, welches jeweils gelenkig mit dem Gelenkteil (6,9) des oberen und unteren Implantatteils (2,3) zusammenwirkt.

13. Implantat nach Anspruch 12, **dadurch gekennzeichnet, dass** das Zwischenbauteil (11) von dem Verbindungsmittel (4) durchgriffen wird.

14. Implantat nach einem der Ansprüche 1-13, **dadurch gekennzeichnet, dass** die Gelenkverbindung zwischen den Implantatteilen (2,3) derart ausgebildet ist, dass beide Gelenkteile (6,9) aufeinander oder auf einem zwischen diesen angeordneten Zwischenbauteil (11) abwälzbar oder seitlich gegeneinander oder gegen das Zwischenbauteil (11) unter Ausbildung der Gelenkverbindung verkippbar sind.

15. Implantat nach einem der Ansprüche 1-14, **dadurch gekennzeichnet, dass** die Gelenkteile (6,9) des oberen und/oder des unteren Implantatteils (2,3) durch das Verbindungsmittel (11) zu einer zusammenhängend handhabbaren Baugruppe verbindbar sind, welche bei jeweils an diesem angeordnetem Verbindungsmittel (4) an dem oberen und/oder unteren Implantatteil (2,3) befestigbar sind.

16. Implantat nach einem der Ansprüche 1-15, **dadurch gekennzeichnet, dass** das Verbindungsmittel (4) oder ein Teil (4a,4b) desselben von der Seite des Anlageteils (5,8) her an dem Gelenkteil (6,9) ankoppelbar ist und/oder das Verbindungsmittel (4) von der Seite des zu verbindenden Knochens her unverlierbar an dem dieses aufnehmenden Gelenkteils (6,9) ankoppelbar ist oder zusammenfügbar ist.

## Claims

1. A joint implant, comprising an upper and a lower implant part (2, 3), which are connected to each other by at least one joint, wherein the upper implant part (2) can be placed and supported with an upper side (2a), and the bottom implant part (3) with a bottom side (3a), against bones to be connected to each other in an articulated manner, wherein the upper and/or the lower implant part (2, 3) respectively comprise joint regions (7, 10) providing a portion of the joint, and wherein a connecting means (4) is provided which connects the upper and lower implant part (2, 3) to each other to form a contiguous assembly, while enabling movement of the joint, **characterized in that** the upper and/or lower implant part (2, 3) respectively comprises a contact part (5, 8) which faces the respective bone and can be attached thereto, and which provides the upper or lower side of the implant, and further comprises a joint part (6, 9) which can be attached to the contact part (5, 8) and comprises the joint region (7, 10) of the implant part, and the connecting means (4) extends substantially parallel to the longitudinal axis (22) of the implant and is retained in a retainer (26, 27) of the joint region (7, 10) of the upper and/or lower implant part (2, 3), said holder being open on the side facing the bone, and a cover element is provided which can be displaced transversely to the longitudinal axis (22) of the implant and which covers the retainer (26, 27) at least partially or at least substantially, and the cover element is arranged as a contact part (5, 8) which can be placed on the bone and supports the implant against the bone.

2. An implant according to claim 1, **characterized in that** the connecting means (4) penetrates the upper and/or lower joint part (6, 9) and secures said part in the longitudinal axis (22) of the implant by absorbing tensile forces.

3. An implant according to claim 1 or 2, **characterized in that** the joint part (6, 9) is attached in an interlocking manner to the contact part (5, 8) of the upper and/or lower implant part (2, 3).

4. An implant according to one of the claims 1 to 3, **characterized in that** the connecting means (4) is displaceable transversely to the longitudinal axis (22) of the implant relative to the upper and/or lower contact part (5, 8) and/or joint part (6, 9).

5. An implant according to one of the claims 1 to 4, **characterized in that** the connecting means (4) comprises two parts (4a, 4b) which can be attached or are attached permanently to each other by formation of a connection in the longitudinal axis (22) of the implant.

6. An implant according to one of the claims 1 to 5, **characterized in that** the implant part (2, 3) and the joint part (6, 9) associated therewith can be attached to each other by an interlocking means (32, 33).

7. An implant according to one of the claims 1 to 6, **characterized in that** the upper and/or lower joint part (6, 9) is formed in a plate-shaped manner and rests on the contact part (5, 8).

8. An implant according to one of the claims 1 to 7, **characterized in that** the upper and/or lower contact part (5, 8) and the associated joint part (6, 9) can respectively be attached to each other in such a way that they can be coupled to each other at least substantially by displacement transversally to the longitudinal axis of the implant.

9. An implant according to claim 8, **characterized in that** the contact part (5, 8) and/or the joint part (6, 9) have an axial guide, along which the two components, guided on each other, are displaceable to their target position with respect to each other.

10. An implant according to one of the claims 1 to 9, **characterized in that** the contact part (5, 8) or the joint part (6, 9) comprises a fastening means which can be attached to the respective other component, and the fastening region of one of the two components bridges the respective other one at least in part by forming a pocket-like receptacle.

11. An implant according to one of the claims 1 to 10, **characterized in that** the contact part (5, 8) and the associated joint part (6, 9) are formed in such a way that they retain the connecting means (4) in a captive fashion when attaching the joint part (6, 9) in its target position to the contact part (5, 8).

12. An implant according to one of the claims 1 to 11, **characterized in that** an intermediate component (11) is provided between the joint part (6, 9) of the upper and lower implant part (2, 3), which intermediate component respectively interacts in an articulated manner with the joint part (6, 9) of the upper and lower implant part (2, 3).

13. An implant according to claim 12, **characterized in that** the intermediate component (1) is penetrated by the connecting means (4).

14. An implant according to one of the claims 1 to 13, **characterized in that** the joint connection between the implant parts (2, 3) is formed in such a way that both joint parts (6, 9) can be rolled off on each other or on an intermediate component (11) arranged between said parts, or can be tilted laterally against each other or against the intermediate component (11) under formation of the joint connection.

15. An implant according to one of the claims 1 to 14, **characterized in that** the joint parts (6, 9) of the upper and/or lower implant part (2, 3) can be connected by the connecting means (11) into an assembly which can be handled contiguously and which can be attached to the upper and/or lower implant part (2, 3) when the connecting means (4) is arranged thereon.

16. An implant according to one of the claims 1 to 15, **characterized in that** the connecting means (4), or a part (4a, 4b) thereof, can be coupled from the side of the contact part (5, 8) to the joint part (6, 9), and/or the connecting means (4) can be coupled or joined in a captive manner from the side of the bone to be connected to the joint part (6, 9) receiving said bone.

## Revendications

1. Implant articulaire avec une partie d'implant supérieure et une partie inférieure (2, 3), qui sont reliées entre elles au moyen d'au moins une articulation, la partie d'implant supérieure (2) pouvant être posée et appuyée par une face supérieure (2a) et la partie d'implant inférieure (3) par une face inférieure (3a) sur des os à relier entre eux de façon articulée, les partie d'implant supérieure et/ou inférieure (2, 3) présentant chacune une partie des régions articulaires (7, 10) formant l'articulation et un moyen de liaison (4) étant prévu, qui relie les partie d'implant supérieure et inférieure (2, 3) en formant un sous-ensemble d'un seul tenant et en permettant un mouvement articulé entre elles, **caractérisé en ce que** les partie d'implant supérieure et/ou inférieure (2, 3) présentent chacune une partie d'appui (5, 8) tournée vers l'os correspondant et pouvant être fixée à celui-ci, qui constitue la face supérieure ou respectivement inférieure de l'implant, et une partie articulaire (6, 9) pouvant être fixée à la partie d'appui (5, 8), qui comprend la région articulaire (7, 10) de la partie d'implant, et **en ce que** le moyen de liaison (4) est retenu en s'étendant de façon sensiblement parallèle à l'axe longitudinal de l'implant (22) dans un réceptacle (26, 27) ouvert du côté de l'os de la région articulaire (7, 10) des parties d'implant supérieure et/ou inférieure (2, 3), et **en ce qu'**il est prévu un élément de couverture qui peut être déplacé perpendiculairement à l'axe longitudinal de l'implant (22) et qui recouvre au moins partiellement ou au moins sensiblement complètement le réceptacle (26, 27), et **en ce que** l'élément de couverture est conformé comme une partie d'appui (5, 8) pouvant être posée sur l'os et appuyant l'implant contre l'os.

2. Implant selon la revendication 1, **caractérisé en ce que** le moyen de liaison (4) traverse la partie d'articulation supérieure et/ou inférieure (6,9) et fixe celle-ci en absorbant la force de traction sur l'axe longitudinal de l'implant (22).

3. Implant selon la revendication 1 ou 2, **caractérisé en ce que** la partie d'articulation (6, 9) est fixée en engagement positif sur la partie d'appui (5, 8) de la partie d'implant supérieure et/ou inférieure (2, 3).

4. Implant selon l'une des revendications 1 à 3, **caractérisé en ce que** le moyen de liaison (4) peut être déplacé par rapport à la partie d'appui supérieure et/ou inférieure (5, 8) et/ou à la partie d'articulation (6, 9) perpendiculairement à l'axe longitudinal de l'implant (22).

5. Implant selon l'une des revendications 1 à 4, **caractérisé en ce que** le moyen de liaison (4) présente deux parties (4a, 4b) qui peuvent être fixées ou sont fixées de façon permanente l'une à l'autre en formant une liaison dans l'axe longitudinal de l'implant (22).

6. Implant selon l'une des revendications 1 à 5, **caractérisé en ce que** la partie d'implant (2, 3) et la partie d'articulation (6, 9) qui lui est associée peuvent être fixées l'une à l'autre par des moyens d'engagement positif (32, 33).

7. Implant selon l'une des revendications 1 à 6, **caractérisé en ce que** la partie d'articulation supérieure et/ou inférieure (6, 9) est en forme de plaque et s'appuie sur la partie d'appui (5, 8).

8. Implant selon l'une des revendications 1 à 7, **caractérisé en ce que** la partie d'appui supérieure et/ou inférieure (5, 8) et la partie d'articulation (6, 9) correspondante peuvent être fixées l'une à l'autre de telle manière qu'elles peuvent être couplées l'une à l'autre au moins pour l'essentiel par translation perpendiculairement à l'axe longitudinal de l'implant.

9. Implant selon la revendication 8, **caractérisé en ce que** la partie d'appui (5, 8) et/ou la partie d'articulation (6,9) présentent un guidage axial le long duquel les deux pièces peuvent être guidées par translation l'une vers l'autre dans leur position de consigne.

10. Implant selon l'une des revendications 1 à 9, **caractérisé en ce que** la partie d'appui (5, 8) ou la partie d'articulation (6, 9) présente un moyen de fixation pouvant être fixé à l'autre pièce et la zone de fixation de chacune des deux pièces recouvre au moins partiellement l'autre en formant un réceptacle en forme de poche.

11. Implant selon l'une des revendications 1 à 10, **caractérisé en ce que** la partie d'appui (5, 8) et la partie d'articulation (6, 9) correspondante sont conformées de telle sorte qu'elles retiennent le moyen de liaison (4) de façon imperdable lorsque la partie d'articulation (6, 9) est fixée dans sa position de consigne sur la partie d'appui (5, 8).

12. Implant selon l'une des revendications 1 à 11, **caractérisé en ce qu'**il est prévu entre les parties d'articulation (6, 9) des parties d'implant supérieure et inférieure (2, 3) une pièce intermédiaire (11) qui coopère de façon articulée avec les parties d'articulation (6, 9) des parties d'implant supérieure et inférieure (2, 3).

13. Implant selon la revendication 12, **caractérisé en ce que** la pièce intermédiaire (11) est traversée par le moyen de liaison (4).

14. Implant selon l'une des revendications 1 à 13, **caractérisé en ce que** la liaison articulée entre les parties d'implant (2, 3) est conformée de telle manière que les deux parties d'articulation (6, 9) puissent rouler l'une sur l'autre ou sur une pièce intermédiaire (11) disposée entre elles ou basculer latéralement l'une vers l'autre ou vers la pièce intermédiaire (11) en formant la liaison articulée.

15. Implant selon l'une des revendications 1 à 14, **caractérisé en ce que** les parties d'articulation (6, 9) des parties d'implant supérieure et/ou inférieure (2, 3) peuvent être reliées par le moyen de liaison (11) pour former un sous-ensemble pouvant être manipulé d'un seul tenant, qui peut être fixé sur la partie d'implant supérieure et/ou inférieure (2, 3) quand le moyen de liaison (4) est disposé respectivement sur celle-ci.

16. Implant selon l'une des revendications 1 à 15, **caractérisé en ce que** le moyen de liaison (4) ou une partie (4a, 4b) de celui-ci peut être couplé à la partie d'articulation (6, 9) à partir du côté de la partie d'appui (5, 8) et/ou le moyen de liaison (4) peut être couplé ou assemblé à partir du côté de l'os à assembler, de façon imperdable, sur la partie d'articulation (6, 9) qui le reçoit.
